Europäisches Patentamt

European Patent Office

Office européen des brevets

(19)

(11) Publication number: **0 348 958**
**A2**

## EUROPEAN PATENT APPLICATION

(21) Application number: 89111804.4

(22) Date of filing: 29.06.89

(51) Int. Cl.4: **C12N 9/16 , C07K 7/00 ,**
**C12N 15/55 , C12N 5/00 ,**
**A01H 1/00**

Claim for the following Contracting State: ES

(30) Priority: 01.07.88 US 213943
13.01.89 US 297003

(43) Date of publication of application:
03.01.90 Bulletin 90/01

(84) Designated Contracting States:
AT BE CH DE ES FR GB GR IT LI LU NL SE

(71) Applicant: THE GENERAL HOSPITAL
CORPORATION
55 Fruit Street
Boston MA 02114(US)

(72) Inventor: Smith, John A.
19 Thatcher Street
Brookline, MA 02146(US)
Inventor: Lee, Fang-Jen S.
33 Day Street No. D
Somerville, MA 02144(US)

(74) Representative: Klein, Otto, Dr. et al
Hoechst AG Zentrale Patentabteilung
Postfach 80 03 20
D-6230 Frankfurt am Main 80(DE)

(54) **Purification and characterization of an acetyl-CoA hydrolase.**

(57) This invention is directed to the isolation, identification, purification and characterization of acetyl-CoA hydrolase, the enzyme having a molecular weight of about 64,000 daltons and an enzyme activity of greater than 20 wherein one unit of acetyl CoA hydrolase activity is defined as the amount of enzyme able to inhibit 1 unit of acetyltransferase activity wherein 1 unit of acetyltransferase activity is defined as the amount of enzyme able to transfer 1 pmol of [$^3$H] acetyl group from [$^3$H] acetyl coenzyme A to ACTH (1-24) by acetyltransferase standard enzyme assay under standard conditions. This invention is further directed to a method for purifying the acetyl-CoA hydrolase.

EP 0 348 958 A2

## PURIFICATION AND CHARACTERIZATION OF AN ACETYL-CoA HYDROLASE

### Field of the Invention

This invention is directed to the isolation, purification, and characterization of acetyl-CoA hydrolase and to the enzyme itself.

## BACKGROUND OF THE INVENTION

Although acetyl-CoA is mainly oxidized in the Krebs cycle or converted to fatty acids or ketone bodies, a varying percentage can be used as donor substrates in co-translational, post-translational, or chemical modification: acetylation. The covalent attachment of an acetyl group to biological substances (e.g., protein, glucosamine, choline, etc.) (Tsunasawa, S., et al., Methods Enzymol. 106:165-170 (1984); Driessen, H.P.C., et al., CRC Crit. Rev. Biochem. 18:281-325 (1985); Klein, U., et al., Proc. Natl. Acad. Sci. U.S.A. 75:5185-5189 (1978); Roskoski, R., Jr., J. Biol. Chem. 249:2156-2159 (1974)) or to chemical compounds (e.g., arylamine, arylalkyamine, etc.) (Jencks, W.P, et al., J. Biol. Chem. 247:3756-3760 (1972); Deguchi, T., et al., J. Biol. Chem. 263:7528-7533 (1988)) is a common chemical modification in the biological system. Acetylation is mediated by acetyl-transferases, which catalyze the transfer of an acetyl group from acetyl coenzyme A to the -NH2 group or -OH group of the biological substances.

The concentration of acetyl-CoA in cells in regulated by its rate of synthesis and its rate of utilization and/or degradation. Acetyl-CoA is primarily synthesized from pyruvate generated from the carbohydrate and from amino acids (Ala, Thr, Gly, Ser, and Cys), from acetoacetyl-CoA generated from other amino acids (Phe, Tyr, Leu, Lys, and Trp), and from $\beta$-oxidation of fatty acids, although a minor amount is also synthesized by acetyl-CoA synthetase (Frenkel, E.P. et al., J. Biol. Chem. 252:504-507 (1977)). Utilization of acetyl-CoA occurs during the Krebs cycle or by its conversion to fatty acids or ketone bodies. Furthermore, the acetyltransferase-catalyzed acetylation of proteins and peptides (Tsunasawa, S. et al., Methods Enzymol. 106:165-170 (1984); Driessen, H.P.C. et al., CRC Crit. Rev. Biochem. 18:281-325 (1985); Persson, B. et al., Eur. J. Biochem. 152:523-527 (1985); Augen, J. et al., Trends Biochem. Sci. 11:494-497 (1986); Allfrey, V.G. et al., Methods Enzymol 107:224-240 (1984); Rudman, D. et al., J. Biol. Chem. 254:10102-10108 (1979)), as well as of biological substances other than proteins (e.g., glucosamine, choline, arylamine, arylalkylamine) (Klein, U. et al., Proc. Natl. Acad. Sci. USA 75:5185-5189 (1978); Roskoski, R., Jr., J. Biol. Chem. 249:2156-2159 (1974); Jencks, W.P. et al., J. Biol. Chem. 247:3756-3760 (1972); Weber, W.W. et al., Pharmacol. Rev. 37:25-79 (1985) accounts for additional usage of endogenous acetyl-CoA.

An acetyl-CoA hydrolase (EC 3.1.2.1) hydrolyzes acetyl-CoA to acetate and CoA. This enzyme was first found in pig heart in 1952 (Gergely, J., et al., J. Biol. Chem. 263:313-319 (1952)) and subsequently has been found in many mammalian tissues (Knowles, S.E., et al., Biochem. J. 142:401-411 (1974); Robinson, J.B., et al., Biochem. Biophys. Res. Commun. 71:959-965 (1976); Bernson, V.M.S., Eur. J. Biochem. 67:403-410 (1976); Grigat, K.P., et al., Biochem. J. 177:71-79 (1979); Prass, R.L., et al., J. Biol. Chem. 255:5215-5223 (1980); Soling, H.D., et al., Eur. J. Biochem. 147:111-117 (1985)). In rat liver, two isoenzymes of acetyl-CoA hydrolase have been found. One is located in the matrix space of mitochondria (Soling, H.D., et al., Eur. J. Biochem. 147:111-117 (1985)) and another in the cytoplasm (Prass, R.L., et al., J. Biol. Chem. 255:5215-5223 (1980)). Although only the rat liver cytoplasmic enzyme has been purified to homogeneity (Prass, R.L. et al., J. Biol. Chem. 255:5215-5223 (1980)), the rat brain mitochondrial enzyme has also been partially purified and characterized (Robinson, J.B. et al., Biochem. Biophys. Res. Commun. 71:959-965 (1976)). The soluble cytosolic acetyl-CoA hydrolase found in rat liver is cold labile, inhibited by 5'ADP, but activated by 5'ATP (Prass, R.L., et al., J. Biol. Chem. 255:5215-5223 (1980)). However, the mitochondrial acetyl-CoA hydrolase is not affected by cold temperature (4°C) and these nucleotides (Soling, H.D., et al., Eur. J. Biochem. 147:111-117 (1985)). CoASH is a strong product inhibitor of the cold-labile enzyme, but only a weak inhibitor of the mitochondrial enzyme (Soling, H.D., et al., Eur. J. Biochem. 147:111-117 (1985)).

Although there have been many studies on the physicochemical properties of acetyl-CoA hydrolase, little is known about its biological meaning. It has been proposed that a physiological role of acetyl-CoA hydrolase may be the maintenance of the cytosolic acetyl-CoA concentration and CoASH pool for both fatty acid synthesis and oxidation (Prass, R.L. et al., J. Biol. Chem. 255:5215-5223 (1980)). Bernson (Eur. J. Biochem. 67:403-410 (1976)) has also suggested that the enzyme in hamster brown adipose may be involved in the post-hibernation warming-up process. Moreover, the Klein group (Namboodiri, M.A.A. et al., J. Biol. Chem. 255:6032-6035 (1980)) has found that arylalkylamine N-acetyltransferase in the rat pineal gland plays a key role in generation of the circadian rhythmicity of melatonin synthesis and suggested that acetyl-CoA hydrolase may be involved in the regulation of acetylation process. The involvement of acetyl-CoA hydrolase in regulating the endogenous pool(s) of acetyl-CoA, and functioning as the donor of acetyl groups during protein synthesis has not yet been established.

Acetyl-CoA hydrolase has been found to be an important contaminant inhibitor of purified rat brain pyruvate carboxylase (Mahan, D.E., et al., Biochem. J. 145:25-35 (1974)) and choline acetyltransferase (Stverin, S.E., et al., Biokhimia 32:125-131 (1967)).

An inhibitor of acetyltransferase has been previously demonstrated in a crude homogenate from pituitary (Glembotski, C.C., J. Biol. Chem. 257:10501-10509 (1982)) and a crude lysate from yeast (Travis, G.H., et al., J. Biol. Chem.259:14406-14412 (1984)). This inhibitor has been suspected to be the acetyl-CoA hydrolase (Dixon, J.E., et al., Methods Enzymol. 106:170-179 (1984)). In addition, acetyl-CoA hydrolase has been found to inhibit purified rat brain pyruvate carboxylase (Mahan, D.E. et al., Biochem. J. 145:25-35 (1975)), choline acetyltransferase (Severin, S.E. et al., Biokhimia 32:125-131 (1967)), and [acyl-carrier-protein] acetyltransferase (Lowe, P.N. et al., Biochem. J. 250:789-796 (1988)).

Given the potential biochemical importance of acetyl-CoA hydrolase, it is of interest to examine the subcellular location, substrate specificity, and regulation of the enzyme. In order to cast light on the biological implications of the hydrolysis and to elucidate the enzymatic mechanism of the reaction, as a first step, the enzyme must be isolated, and then purified and characterized.

## SUMMARY OF THE INVENTION

According to this invention, an acetyl-CoA hydrolase which hydrolyzes acetyl-CoA to acetate and CoASH was isolated and purified to homogeneity. The invention therefore relates to the purification to homogeneity of acetyl-CoA hydrolase and to the purified acetyl-CoA hydrolase.

Acetyl-CoA hydrolase, which hydrolyzes acetyl-CoA to acetate and CoASH, was isolated from Saccharomyces cerevisiae and demonstrated by protein sequence analysis to be $NH_2$-terminally blocked. The enzyme was purified 2400-fold to apparent homogeneity by successive purifica tion steps using DEAE-Sepharose, gel filtration, and hydroxyapatite. The $M_r$ of the active yeast acetyl-CoA hydrolase was estimated to be 64,000 ± 5,000 by gel filtration chromatography. SDS-PAGE analysis revealed that the denatured molecular weight was 65,000 ± 2,000 and together with $M_r$ for the native enzyme indicate that yeast acetyl-CoA hydrolase was monomeric. The enzyme had a pH optimum near 8.0, and its pI was ~ 5.8. Several acyl-CoA derivatives of varying chain length were tested as substrates for yeast acetyl-CoA hydrolase. Although acetyl-CoA hydrolase was relatively specific for acetyl-CoA, longer acyl-chain CoAs were also hydrolyzed and were capable of functioning as inhibitors during the hydrolysis of acetyl-CoA. Among a series of divalent cations, $Zn^{2+}$ was demonstrated to be the most potent inhibitor. The enzyme was inactivated by chemical modification with diethyl pyrocarbonate, a histidine-modifying reagent.

## BRIEF DESCRIPTION OF THE FIGURES

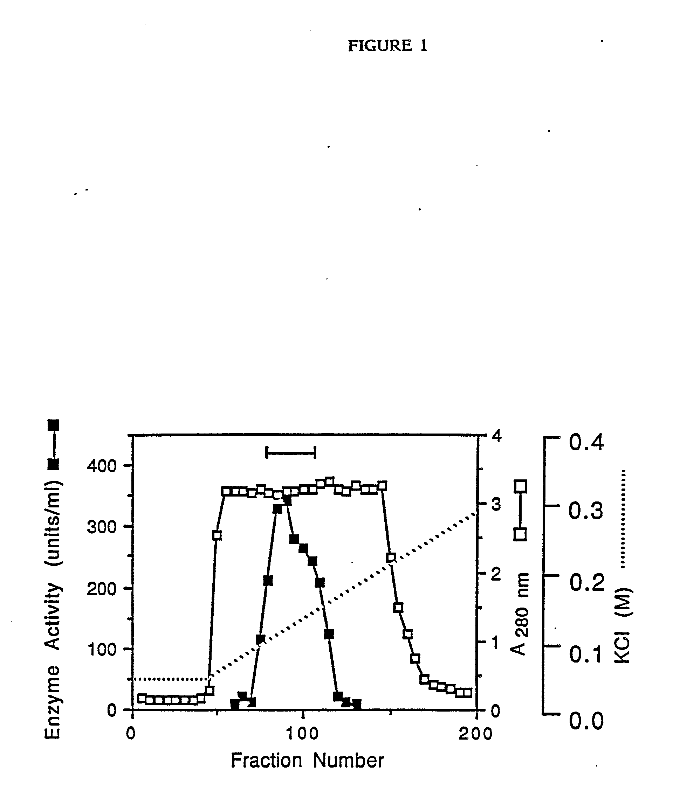

Figure 1 shows DEAE-Sepharose (0.05 to 0.5 M KCl) chromatography of the yeast acetyl-CoA hydrolase. The acetyl-CoA hydrolase pool, which also contained $N^{\alpha}$-acetyltransferase (Lee, F.J.S., et al., J. Biol. Chem. 263:14948-14955 (1988)), from DEAE-Sepharose (0.2 M KCl) (as described below) was concentrated, dialyzed, and applied to a DEAE-Sepharose column, eluted with a linear gradient of 0.05 M (250 ml) to 0.5 M (250 ml) KCl in HDG buffer at 24 ml/h, and analyzed for $A_{280}$, conductivity, and enzyme activity (acetylation inhibition assay), as described below. Fractions containing acetyl-CoA hydrolase activity were pooled as indicated by the horizontal bar.

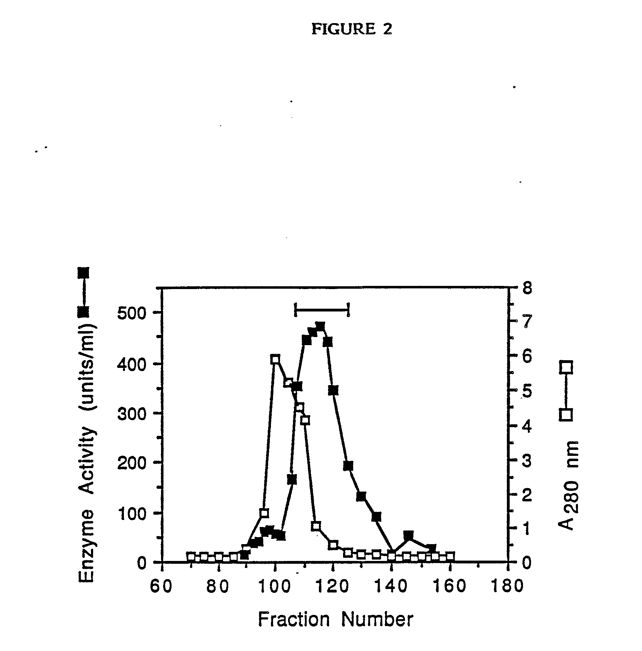

Figure 2 shows Sepharose CL-4B chromatography of the yeast acetyl-CoA hydrolase. The acetyl-CoA hydrolase pool from DEAE-Sepharose (0.05 to 0.5 M KCl) was concentrated and applied to a Sepharose CL-4B gel column, eluted with 200 mM potassium phosphate buffer, pH 7.4, containing 0.5 mM DTT, 10% (v/v) glycerol, 0.02% $NaN_3$ at 20 ml/h, and analyzed for $A_{280}$ and enzyme activity (acetylation inhibition assay), as described below. Fractions containing acetyl-CoA hydrolase activity were pooled as indicated by the horizontal bar.

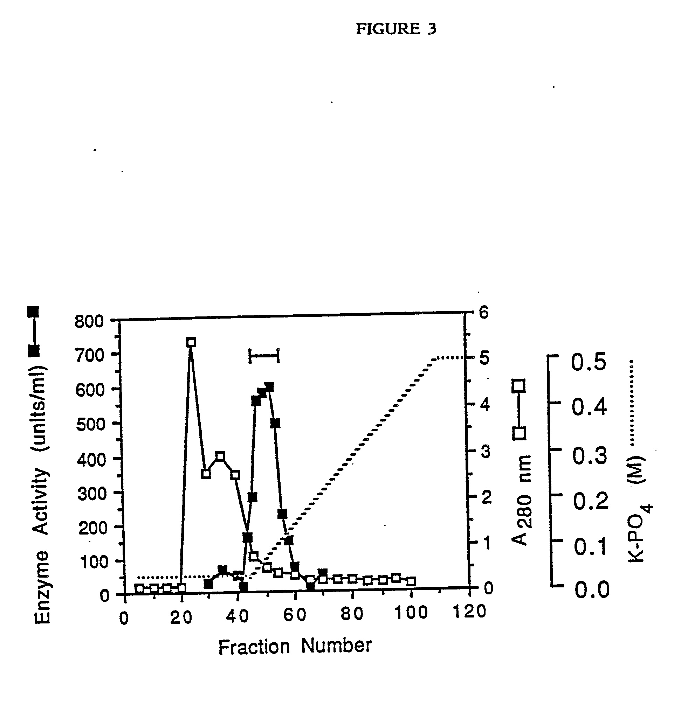

Figure 3 shows Hydroxylapatite chromatography analysis of the yeast acetyl-CoA hydrolase. The acetyl-CoA hydrolase pool from Sepharose CL-4B chromatography was concentrated, dialyzed, and applied to a hyroxylapatite column, eluted with a linear gradient of 0.05 M (150 ml) to 0.5 M (150 ml) potassium phosphate buffer, pH 7.4, containing 0.5 mM DTT, 10% (v/v) glycerol, 0.02% NaN$_3$ at 12 ml/h, and analyzed from A$_{280}$, conductivity, and enzyme activity (acetylation inhibition assay), as described below. Fractions containing acetyl-CoA hydrolase activity were pooled as indicated by horizontal bar.

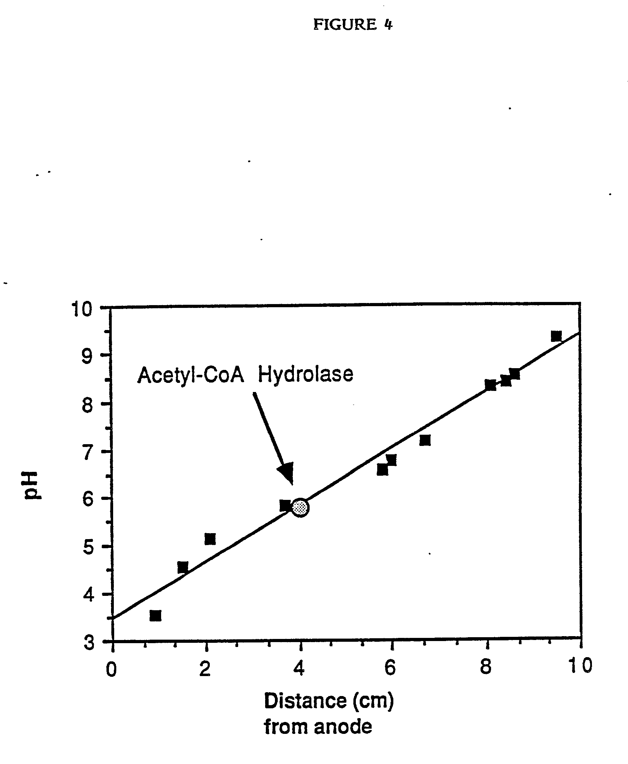

Figure 4 shows a determination of isoelectric point of yeast acetyl-CoA hydrolase by isoelectric focusing. Isoelectric focusing, using an aliquot from the hydroxylapatite pool (Figure 3), was performed as previously described (Catsimpoolas, N., Isoelectric Focusing, Academic Press, New York (1976)) (pH range 3.5-9.5). For determination of pI of purified enzyme subunits, A. oryzae amyloglucosidase (pI = 3.55), soybean trypsin inhibitor (pI = 4.55), milk β-lactoglobulin (pI = 5.13), bovine erythrocytes carbonic anhydrase B (pI = 5.85), human erythrocytes carbonic anhydrase B (pI = 6.57), horse heart myoglobin (pI = 6.76 and 7.16), rabbit muscle L-lactic dehydrogenase (pI = 8.3, 8.4, and 8.55), and bovine pancreas trypsinogen (pI = 9.3) were used as pI standards. Protein bands were stained with Coomassie Blue.

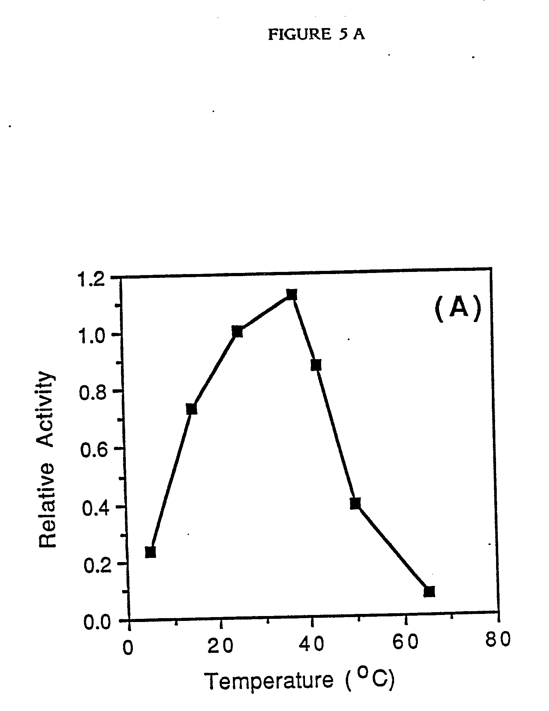

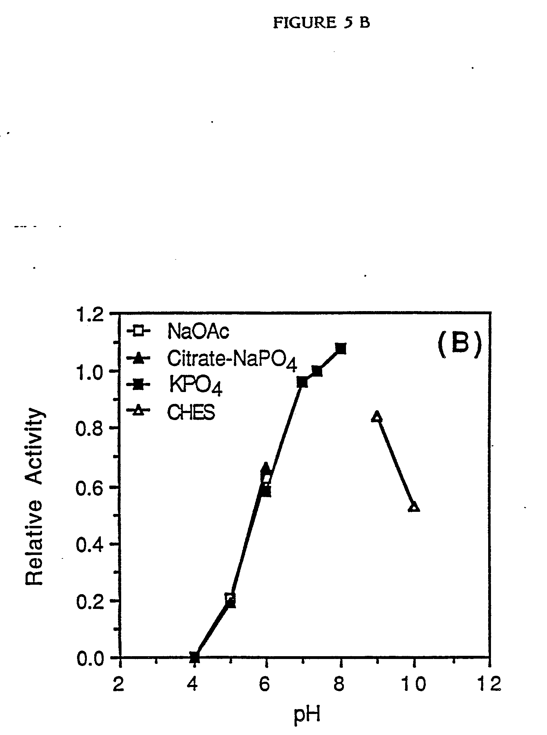

Figure 5 shows the effect of temperature and pH on yeast acetyl-CoA hydrolase. (A) The activity of yeast acetyl-CoA hydrolase for acetyl-CoA was determine at different temperatures by the radioactive assay, as described below. (B) The activity of yeast acetyl-CoA hydrolase for acetyl-CoA was determined in 100 mM buffers of sodium acetate, citrate-phosphase, potassium phosphase, and CHES buffers of different pH by the radioactive assay, as described below.

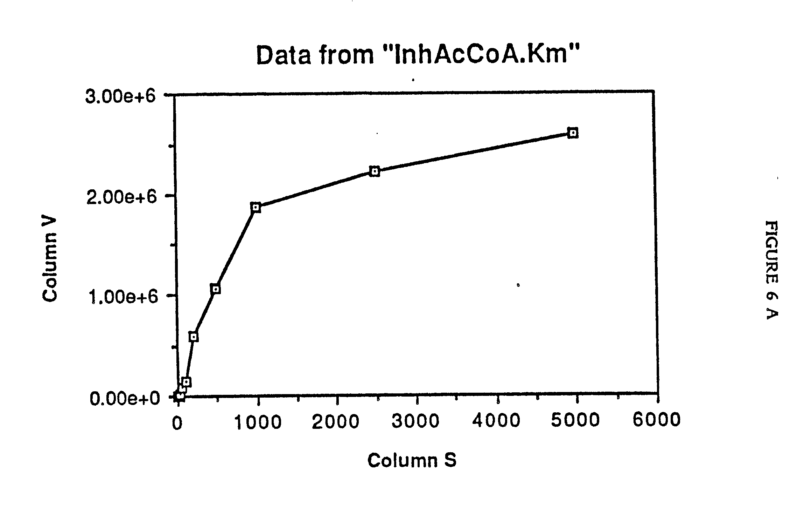

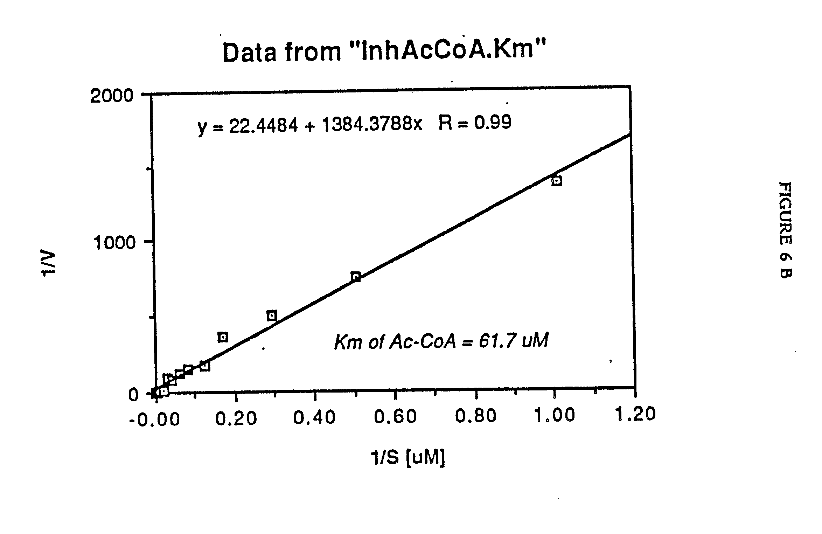

Figure 6 shows the Acetyl-CoA concentration versus rate of acetate releasing for acetyl-CoA hydrolase. (A) straight plot. (B) Lineweaver-Burk plot.

## DETAILED DESCRIPTION OF THE INVENTION

Acetyl-CoA hydrolase is an enzyme which hydrolyzes acetyl-CoA to acetate and coenzyme A (CoA). During the purification of yeast N$^{\alpha}$-acetyltransferase, the inventors detected the presence of an endogenous inhibitor of acetyltransferase as evidenced by the 204% recovery of the enzyme activity observed after the DEAE-Sepharose chromatography (United States Patent Application Serial No. 153,361, filed February 8, 1988). The biological activity of the inhibitor was then characterized, resulting in the identification of the inhibitor as an acetyl-CoA hydrolase (Lee, F.J.S. et al., J. Biol. Chem. 263:14948-14955 (1988), which reference is herein incorporated by reference in its entirety). In order to elucidate the functional role of this enzyme in the acetylation process, this enzyme was subsequently purified to homogeneity by a novel assay and its substrate specificity investigated using a series of acyl-CoA.

### I. Isolation, Identification, Purification, and Characterization of Acetyl-CoA Hydrolase

In accordance with this invention, the acetyl-CoA hydrolase can be isolated from a yeast sample containing the enzyme. Any yeast sample that contains the enzyme may be used as a starting material according to the methods described in this invention. As used herein, the sample containing the enzyme will be referred to simply as "sample", which is intended to include acetyl-CoA hydrolase-containing yeast sample.

Although others have attempted to isolate this enzyme from a variety of sources including rat brain (Robinson, J.B., et al., Biochem. Biophys. Res. Commun. 71:959-965 (1976)), rat liver (Prass, R.L., et al., J. Biol. Chem. 255:5215-5223); Soling, H.D., et al., Eur. J. Biochem. 147:111-117 (1985)), and hamster brown adipose tissue (Bernson, V.M.S., Eur. J. Biochem. 67:403-410 (1976)), only the cytosolic enzyme from rat liver (Prass, R.L., et al., J. Biol. Chem. 255:5215-5223 (1980)) has been purified. The properties of this yeast acetyl-CoA hydrolase of this invention are very different from that previously purified by Prass et al., supra, from rat liver.

### Homogeneity and Molecular Properties.

An acetyl-CoA hydrolase from S. cerevisiae was purified 2,400-fold fro 800 g of cells (Table 1) with an overall recovery of 31%, by successive chromatographic procedures utilizing DEAE-Sepharose, Sepharose CL-4B, and hydroxylapatite. The enzyme comprises approximately 0.2% of the total cellular protein.

4

## Table 1

## Purification of Acetyl-CoA Hydrolase from S. cerevisiae

| Step | Activity[a] (units) | Protein (mg) | Specific Activity (unit/mg) | Purification (fold) | Yield (%) |
|---|---|---|---|---|---|
| 1. Crude Extract | 31300[b] | 19500 | 1.6 | 1.0 | 100[b] |
| 2. DEAE-Sepharose (0.2 M KCl) | 31400[b] | 4250 | 7.4 | 4.6 | 100[b] |
| 3. DEAE-Sepharose (0.05 - 0.5 M KCl) | 26300 | 862 | 30.5 | 19.1 | 84 |
| 4. Sepharose CL-4B | 16800 | 304 | 55.3 | 34.6 | 54 |
| 5. Hydroxylapatite | 9600 | 2.5 | 3840 | 2400 | 31 |

a   Activity was measured as described below.
b   In the first two chromatographic steps, $N^\alpha$-acetyltransferase and acetyl-CoA hydrolase were present in the same fractions (Lee, F.J.S. et al., J. Biol. Chem. 263:14948-14955 (1988)), and the activity of acetyl CoA hydrolase could not be determined. After the third chromatographic step, the $N^\alpha$-acetyltransferase and acetyl CoA hydrolase were separated, and the total activity of $N^\alpha$-acetyltransferase was 63,300 units. However, the activity of $N^\alpha$-acetyltransferase determined in the first two steps was only 32,000 units. If one assumes that the true activity of $N^\alpha$-acetyltransferase in the first two chromatographic steps was actually 63,300 units, and that all the inhibition of acetyltransferase was caused by acetyl CoA hydrolase, then the activity of acetyl CoA hydrolase can be estimated to be 31,300 units in each of the first two chromatographic steps.

The molecular weight of the acetyl-CoA hydrolase was determined using gel electrophoresis. An aliquot of sample was analyzed using a 9% gel according to the method of Laemmli (Laemmli, U.K., Nature 227:680-685 (1970)) and compared to molecular weight standards: myosin (205,000), E. coli β-galactosidase (116,000), rabbit muscle phosphorylase (97,000), bovine serum albumin (66,000), and egg albumin (45,000). SDS-PAGE revealed a single Coomassie blue-stained band with an $M_r = 65,000 \pm 2,000$.

The molecular weight of native yeast acetyl-CoA hydrolase was estimated by gel filtration. An aliquot of the purified enzyme from hydroxylapatite chromatography was applied to a Sepharose CL-4B column (2.5 x 96 cm). The elution buffer was HDG buffer containing 0.2 M KCl, and the flow rate was 20 ml/h as described below. The elution volume of acetyl-CoA hydrolase was determined by the acetylation inhibition assay, and the apparent molecular weight was calculated from the relative elution volumes of protein standards including (1) thyroglobulin (669,000), (2) apoferritin (443,000), (3) β-amylase (200,000), (4) alcohol dehydrogenase (150,000), (5) bovine serum albumin (66,000), and (6) carbonic anhydrase (29,000). Gel filtration chromatography on Sepharose CL-4B showed that the $M_r$ of the native acetyl-CoA hydrolase is 64,000±5,000. These data indicate that he yeast acetyl-CoA hydrolase is monomeric.

Isoelectric focusing revealed a single protein band at apparent pI = 5.8 (Figure 4). Assays for determining the temperature optimum for yeast acetyl-CoA hydrolase were performed from 5 to 65° C (Figure 5A).

The pH dependence of yeast acetyl-CoA hydrolase was measured by assaying at pH values from 4 to 10 in the presence of 100 mM buffers of sodium acetate, citrate phosphate, potassium phosphate, and CHES buffers (Figure 5B). Maximum enzyme activity is observed at pH 8 but is reduced to <20% below pH 5.0 and <10% above pH 10.

Assays for determing the temperature optimum for yeast acetyl-CoA hydrolase were performed from 5 to 65°C (Figure 5A), and the yeast acetyl-CoA hydrolase displayed a maximum activity at temperatures from 37 to 42°C. Irreversible denaturation occurred after 1 minute at 65°C. The enzyme was most stable when stored at 4°C in 0.1 M potassium phosphate buffer, pH 7.4. Under these conditions, the purified enzyme had a half-life of approximately 2 weeks. The enzyme was less sensitive to freezing at all stages of purification and displayed only a <10% loss of activity per freeze-thaw cycle.

In Figure 6A, a plot is depicted of the substrate concentration versus reaction velocity for the acetyl-CoA hydrolase assayed with the radioactive assay. The curve has the regular hyperbolic shape which represents an enzyme obeying ordinary Michaelis-Menten kinetics. Figure 6B is a Lineweaver-Burk plot giving the apparent Km for acetyl-CoA to be 62 $\mu$M. Further, the hydrolysis of [1-$^{14}$C] acetyl-CoA by acetyl-CoA hydrolase is inhibited by <30% at CoASH concentrations of >25 $\mu$M.

As used herein, the term "substantially pure" or "substantially purified" is meant to describe acetyl-CoA hydrolase which is substantially free of any compound normally associated with the enzyme in its natural state, i.e., free of protein and carbohydrate components. The term is further meant to describe acetyl-CoA hydrolase which is homogeneous by one or more purity or homogeneity characteristics used by those of skill in the art. For example, a substantially pure acetyl-CoA hydrolase will show constant and reproducible characteristics within standard experimental deviations for parameters such as the following: molecular weight, chromatographic techniques, and such other parameters. The term, however, is not meant to exclude artificial or synthetic mixtures of the enzymes with other compounds. The term is also not meant to exclude the presence of minor impurities which do not interfere with the biological activity of the enzyme, and which may be present, for example, due to incomplete purification.

## Amino Acid Analysis

Samples of electroeluted yeast acetyl-CoA hydrolase were subjected to amino acid analysis, and the amino acid composition of the enzyme is shown in Table 2. The amino acid composition is characteristic of a globular protein.

Table 2

| Amino Acid Composition of Acetyl-CoA Hydrolase from S. cerevisiae [a] | |
|---|---|
| Amino Acid | Observed Residues[b] |
| Asx | 75 |
| Thr | 24 |
| Ser | 33 |
| Glx | 53 |
| Pro | 51 |
| Gly | 34 |
| Ala | 49 |
| Val | 44 |
| Met | 4 |
| Ile | 35 |
| Leu | 51 |
| Tyr | 15 |
| Phe | 37 |
| Lys | 29 |
| His | 17 |
| Arg | 28 |

[a] Purified acetyltransferase was electroeluted from a preparative SDS-PAGE gel. The amino acid composition was determined from six different enzyme preparations using a Beckman 6300 Amino Acid Analyze after 24 hr hydrolysis at $110°$ C in 6 N HCl containing 0.1% phenol. Asx = Asp + Asn; Glx = Glu + Gln.
[b] Residue number per subunit of enzyme was calculated on the basis of a $M_r$ = 64,000. No correction was made for the amounts of Ser and Thr destroyed during the 24 hr hyd olysis. Cys and Trp were not determined.

Amino Acid Sequence Analysis.

Two different preparations of the electroeluted protein (each -300 pmole) were submitted to protein sequence analysis. Except for background levels of amino acids at the first cycle, no protein sequence was detected. These results demonstrate that the N-terminus of the protein is blocked.

However, sequence information was then obtained by cleaving the enzyme with trypsin and then separating the fragments on an HPLC phenyl reverse phase column. Two of the fragments were sequenced. The sequence information for these two random fragments are as follows:

Fragment #689:

```
     2      4      6      8      10     12      14
Phe-Asn-Leu-Phe-Val-Gly-Ala-Ser-Ala-Gly-Pro-Glu-Glu-Asn-(Arg)
```

Fragment #690:

```
     2      4      6      8      10
Val-Val-Ala-Ile-Val-Glu-Ser-Thr-Met-Arg-Leu
```

As detailed below, the sequence information obtained from the random fragments may be used to synthesize oligonucleotide probes which can be used for genetically engineering the enzyme.

Effects of Nucleotides and Divalent Cations on Enzyme Activity.

The effects of various nucleotides and divalent cations on the enzyme activity were determined (Table 3). At 0.1 mM nucleotide, only $\beta$NADH appreciably decreases enzyme activity, and the effect is concentration-dependent. A concentration dependent inhibition occurs in the presence of each divalent cation:
$$(Zn^{2+} > Cd^{2+} = Co^{2+} > Mn^{2+} = Mg^{2+} > Ca^{2+} = Fe^{2+} = Cu^{2+})$$

Table 3

| Effect of Nucleotides and Ions on Enzyme Activity of Acetyl-CoA Hydrolase from S. cerevisiae [a] | | |
|---|---|---|
| Additions | Enzyme Activity (%) | | |
| | Concentration (mM) | | |
| | 10 | 1 | 0.1 |
| None | 100 | 100 | 100 |
| $\beta$NADH | 46 | 69 | 83 |
| $\beta$NAD | 95 | 98 | 100 |
| $\beta$NADPH | 96 | 98 | 99 |
| AMP | 81 | 89 | 96 |
| ADP | 91 | 94 | 98 |
| ATP | 96 | 99 | 101 |
| $ZnSO_4$ | 25 | 88 | -- |
| $CdCl_2$ | 48 | 91 | -- |
| $CoCl_2$ | 51 | 86 | -- |
| $MnCl_2$ | 64 | 84 | -- |
| $MgCl_2$ | 72 | 95 | -- |
| $MgSO_4$ | 75 | 98 | -- |
| $CaCl_2$ | 84 | 97 | -- |
| $FeSO_4$ | 88 | 98 | -- |
| $CuSO_4$ | 89 | 94 | -- |

[a] Yeast acetyl-CoA hydrolase was incubated in the presence of various nucleotides and divalent cations in 100mM potassium phosphate, pH 7.4. The enzyme activity was determined under standard radioacti e assay conditions using ACTH (1-24) (N = 3), as described in the Examples.

Effect of Chemical Modifications on Enzyme Activity.

In order to determine the possible catalytic role for different types of amino acid residues in the enzymes, various chemical modifications were carried out (Table 4). The reaction of acetyl-CoA hydrolase with diethyl pyrocarbonate, a histidine-modifying reagent (Miles, E.W., Methods Enzymol. 47:431-442 (1977)), causes a nearly complete inactivation of the enzyme. After incubation for 8 h at room temperature with 0.25 M hydroxylamine, a reagent capable of reversing the ethoxyformylation of a histidine residue, -55% of the original enzyme activity could be recovered, although prolonged exposure to hydroxyamine slowly inactivated the enzyme. The presence of a catalytically important tryptophan residue was investigated by chemical modification with NBS (Spande, T.F. et al., Methods Enzymol. 11:506-522 (1967)) and HNBS(CH$_3$)$_2$-Br (Horton, H.R. et al., Methods Enzymol. 25:468-482 (1972)). NBS inactivates the enzyme 70% at 0.5 mM and completely inactivated the enzyme at 5 mM. Although HNBS(CH$_3$)$_2$-Br partially inactivates the enzyme, the loss of enzyme activity is small in comparison to NBS. Since NBS can also modify histidine and tyrosine residues (Witkop, B., Adv. Protein Chem. 16:221-321 (1963)), it is possible that the inactivation might be due to a chemical modification of the same histidine(s), presumably modified by diethyl pyrocarbonate. Sulfhydryl-reducing agents (i.e., 2-mercaptoethanol and DTT) do not affect enzyme activity. Sulfhydryl-modifying reagents (i.e., NEM, iodoacetic acid, iodoacetamide, and pCMB) have no effect at 1 mM, but partially inactivate the enzyme at 10 mM. Succinic anhydride and TNBS, which modify lysine residues and $\alpha$-NH$_2$ groups (Riordan, J.F. et al., Methods Enzymol. 25:500-506 (1972); Fields, R., Methods Enzymol. 25:464-468 (1972)), have no effect at 1 mM, but partial inactivation of the enzyme is observed with each reagent at 10 mM. N-acetylimidazole, a tyrosine-modifying reagent, also only inactivates at the higher concentration (Riordan, J.F. et al., Methods Enzymol. 25:500-506 (1972)).

Table 4

| Effect of Protein Modification Reagents on Enzyme Activity of Acetyl-CoA Hydrolase from S. cerevisiae [a] | | |
|---|---|---|
| Reagent Added[b] | Concentration (mM) | Enzyme Activity (%) |
| None | | 100 |
| DEPC | 0.5 | 19 |
| | 5.0 | 3 |
| NBS | 0.5 | 30 |
| | 5.0 | 2 |
| HNBS(CH$_3$)$_2$-Br | 1.0 | 84 |
| | 10.0 | 71 |
| 2-mercaptoethanol | 10.0 | 101 |
| DTT | 10.0 | 104 |
| NEM | 1.0 | 94 |
| | 10.0 | 52 |
| IAA | 1.0 | 99 |
| | 10.0 | 89 |
| IAM | 1.0 | 97 |
| | 10.0 | 85 |
| pCMB | 1.0 | 102 |
| | 10.0 | 88 |
| TNBS | 1.0 | 94 |
| | 10.0 | 77 |
| Succinic anhydride | 1.0 | 95 |
| | 10.0 | 79 |
| N-acetylimidazole | 1.0 | 101 |
| | 10.0 | 67 |

[a] Yeast acetyl-CoA hydrolase was incubated with each reagent at 25° C for 15 min, dialyzed against 100 mM potassium phosphate buffer, pH 7.4, at 4° C for 3 to 4 hr. The enzyme activity was determined un er standard radioactive assay conditions using acetyl-CoA (1-24) (N = 3), assayed as described in the Examples.

[b] Abbreviations: DEPC, diethyl pyrocarbonate; NBS, N-bromosuccinimide; HNBS(CH$_3$)$_2$-Br, dimethyl-(2-hydroxy-5-nitrobenzyl)-sulfonium bromide; NEM, N-ethylmaleimide; IAA, iodoac tic acid; IAM, iodoacetamide; pCMB, p-chloromercuribenzoate; TNBS, 2,4,6-trinitrobenzenesulfonic acid.

Substrate Specificity.

Several acetyl-CoA derivatives of varying chain length were tested as substrates for yeast acetyl-CoA hydrolase. Each of the acetyl-CoAs in Table 5 were compared to acetyl-CoA. Enzyme activities were determined at substrate concentrations of 0.25 mM. Taken together, these results indicate that longer acyl-chain CoAs are also substrates for hydrolysis by acetyl-CoA hydrolase, although the enzyme is relatively specific for acetyl-CoA. Furthermore, each acyl-CoA is capable of functioning as an inhibitor of acetyl-CoA hydrolase, although acetyl-CoA inhibits most effectively (Table 6).

Table 5

| Inhibition of Acyl-CoA on Enzyme Activity of Acetyl-CoA Hydrolase from S. cerevisiae [a] | |
|---|---|
| Substrates | Relative Activity (%) |
| Acetyl-CoA | 100 |
| Malonyl-CoA | 1 |
| Propionyl-CoA | 9 |
| Butyryl-CoA | 4 |
| Acetoacetyl-CoA | 5 |
| Succinyl-CoA | 8 |
| Myristoyl-CoA | 3 |

[a] The enzyme activity was determined using the colorimetric assay (N = 3) described in the Examples.

Table 6

| Inhibition of Acyl-CoA on Enzyme Activity of Acetyl-CoA Hydrolase from S. cerevisiae [a] | | | |
|---|---|---|---|
| Additions | Enzyme Activity (%) | | |
| | Concentration (mM) | | |
| | 5 | 1 | 0.1 |
| None | 100 | 100 | 100 |
| Acetyl-CoA | 5 | 28 | 66 |
| Myristoyl-CoA | 52 | 83 | 93 |
| Succinyl-CoA | 56 | 79 | 92 |
| Butyryl-CoA | 62 | 86 | 95 |
| Propionyl-CoA | 72 | 89 | 92 |
| Acetoacetyl-CoA | 90 | 92 | 98 |
| Malonyl-CoA | 52 | 83 | 93 |

[a] Yeast acetyl-CoA hydrolase was incubated in the presence of various acyl-CoA in 100 mM phosphate buffer, pH 7.4. The enzyme activity was determined under radioactivity assay conditions using acetyl- oA (N = 3), as described in the Examples.

Acetyl-CoA hydrolase, which hydrolyzes acetyl-CoA to acetate and CoASH, was thus isolated from Saccharomyces cerevisiae. During a previous purification of yeast Nα-acetyltransferase, the presence of an endogenous inhibitor of acetyltransferase was demonstrated by the 204% recovery of Nα-acetyltransferase activity after the third chromatographic step, using an assay in which a labelled acetyl group is transferred from [3H] acetyl-CoA to a synthetic peptide, ACTH (1-24) (Lee, F.J.S. et al., J. Biol. Chem. 263:14948-14955 (1988)). Initially, it was not clear whether this inhibition was due to: (i) a protein inhibitor specific for Nα-acetyltransferase; (ii) an acylpeptide hydrolase (Tsunasawa, S. et al., J. Biochem. 77:89-102 (1975); Kobayashi, K. et al., J. Biochem. 262:11435-11445 (1987)); (iii) a thiolase (Kornblatt, J.A. et al., J. Biol. Chem. 246:4417-4423 (1971); Clinkenbeard, K.D. et al., J. Biol. Chem. 248:2275-2284 (1973)); (iv) an acetyl-CoA carrier protein (possibly another acetyltransferase); or (v) an acetyl-CoA hydrolase. After a series of experiments which ruled out the first four mechanisms of inhibition (data not shown), it was determined that inhibition resulted from substrate depletion by an acetyl-CoA hydrolase. Such an enzyme has also been demonstrated to inhibit pyruvate carboxylase from rat brain (Mahan, D.E. et al., Biochem. J. 145:25-35 (1975)), choline acetyltransferase from rabbit brain (Severin, S.E. et al., Biokhimia 32:125-131 (1967)), and [acyl-carrier-protein] acetyltransferase from E. coli (Lowe, P.N. et al., Biochem. J. 250:789-796 (1988)), as well as presumed to inhibit $N^\alpha$-acetyltransferase from a crude homogenate from bovine pituitary (Glembotski, C.C., J. Biol. Chem. 257:10501-10509 (1982)) and rat pituitary (Dixon, J.E. et al., Methods Enzymol. 106:170-179 (1984)) and $N^\alpha$-acetyltransferase from a crude lysate from yeast (Travis, G.H. et al., J. Biol. Chem. 2159:14406-14412 (1984)).

Acetyl-CoA hydrolase was first identified in pig heart in 1952 (Gergely, J. et al., J. Biol. Chem. 198:323-334 (1952)), and subsequently the enzyme has been found in many mammalian tissues (Knowles, S.E. et al., Biochem. J. 142:401-411 (1974); Robinson, J.B. et al., Biochem. Biophys. Res. Commun. 71:959-965 (1976); Bernson, V.S.M., Eur. J. Biochem. 67:403-410 (1976); Grigat, K.P. et al., Biochem. J. 177:71-79 (1979); Prass, R.L. et al., J. Biol. Chem. 255:5215-5223 (1980); Soling, H.D. et al., Eur. J. Biochem. 147:111-117 (1985)). Although others have attempted to isolate acetyl-CoA hydrolase from a variety of sources including: rat brain (Robinson, J.B. et al., Biochem. Biophys. Res. Commun. 71:959-965 (1976)), hamster brown adipose tissue (Bernson, V.S.M., Eur. J. Biochem. 67:403-410 (1976)), and rat liver (Prass, R.L. et al., J. Biol. Chem. 255:5215-5223 (1980); Soling, H.D. et al., Eur. J. Biochem. 147:111-117 (1985)), only the cytosolic enzyme from rat liver (Prass, R.L. et al., J. Biol. Chem. 255:5215-5223 (1980)) was purified previously. We purified yeast acetyl-CoA hydrolase 2,400-fold with an overall recovery of 31% by successive chromatographic procedures utilizing DEAE-Sepharose, Sepharose CL-4B, and hydroxyapatite (Table 1). The $M_r$ of the native yeast acetyl-CoA hydrolase was estimated to be 64,000±5,000 by gel filtration chromatography, and SDS-PAGE revealed that its apparent molecular weight was 65,000±2,000. Hence, native yeast acetyl-CoA hydrolase is monomeric. Although this $M_r$ value for the native yeast enzyme differs from that previously determined for the rat liver cytosolic enzyme by Prass et al. (J. Biol. Chem. 255:5215-5223 (1980)), who determined it to be between 240,000 and 340,000, their estimated subunit $M_r$ of 64,000 is identical to the yeast enzyme. By contrast, the $M_r$ of the native enzyme from rat brain (Robinson, J.B. et al., Biochem. Biophys Res. Commun. 71:959-965 (1976)) or the native mitochrondrial enzyme from rat liver (Soling, H.D. et al., Eur. J. Biochem. 147:111-117 (1985)) is 150,000 and 157,000, respectively. Furthermore, the pH optima for yeast and rat brain mitochondrial acetyl-CoA hydrolase are identical (pH~8) (Robinson, J.B. et al., Biochem. Biophys. Res. Commun. 71:959-965 (1976)). In addition, the yeast enzyme was $NH_2$-terminally blocked, although the chemical nature of this blocking group has not yet been determined.

Two different acetyl-CoA hydrolase have been identified in rat liver. One is located in the cytoplasm, is cold labile, is strongly inhibited by CoASH, is inhibited by ADP and GDP, and is activated by ATP (Prass, R.L. et al., J. Biol. Chem. 255:5215-5223 (1980); Soling, H.D. et al., Eur. J. Biochem. 147:111-117 (1985)). The other is located in the matrix space of mitochondria, is not affected by cold, is only weakly inhibited by CoASH, is not inhibited by ADP or GDP, is not activated by ATP, and is inhibited by NADH (Bernson, V.S.M., Eur. J. Biochem. 67:403-410 (1976); Soling, H.D. et al., Eur. J. Biochem. 147:111-117 (1985)). Yeast acetyl-CoA hydrolase resembles the latter, since it is not cold sensitive, is only weakly inhibited by CoASH, is not affected by ADP or ATP, and is inhibited by $\beta$NADH (Table 3). In addition, the $K_m$ of the yeast acetyl-CoA hydrolase is similar to the mitochondrial enzyme from hamster brown fat (Bernson, V.S.M., Eur. J. Biochem. 67:403-410 (1976)) (62 $\mu$M and 51 $\mu$M, respectively).

Studies using DEPC (and possibly NBS) suggest that a histidine residue may be located within the active site of yeast acetyl-CoA hydrolase, but the failure of $\beta$-mercaptoethanol, DTT, NEM, pCMB, IAA, and IAM to inactivate the enzyme indicates that a cysteine residue is probably not involved in its catalytic mechanism (Table 4).

Although acetyl-CoA hydrolase is relatively specific for acetyl-CoA, longer acyl-chain CoA substrates are also hydrolyzed (Table 5). The functional role of such broad specificity towards various acyl-CoAs is currently not understood. Furthermore, a detailed comparison among the substrate specificities of the yeast enzyme, the non-specific acetyl-CoA hydrolase isolated from pig heart muscle mitochondria (Lee, K.Y. et al., J. Biol. Chem. 254:4516-4523 (1979)), and the short-chain acetyl-CoA hydrolase from brown tissue adipose tissue mitochondria (Alexson, S.E.H. et al., J. Biol. 263:13564-13571 (1988)) has not yet been completed. Furthermore, each acetyl-CoA inhibits yeast acetyl-CoA hydrolase, although acetyl-CoA inhibits most effectively (Table 6).

Acetyl-CoA is primarily synthesized from pyruvate generated from the carbohydrate and from amino acids (Ala, Thr, Gly, Ser, and Cys), from acetoacetyl-CoA generated from other amino acids (Phe, Tyr, Leu, Lys, and Trp), and from $\beta$-oxidation of fatty acids, although a minor amount is also synthesized by acetyl-CoA synthetase (Frenkel, E.P. et al., J. Biol. Chem. 252:504-507 (1977)). Although acetyl-CoA is mainly oxidized in the Krebs cycle or converted to fatty acids or ketone bodies, a varying percentage can be used as a donor substrate for acetyltransferases in co- and post-transnational modifications involving $\alpha$- and $\beta$-

12

NH₂ groups, as well as hydroxyl groups in the seryl and threonyl side chains of protein and peptides. $\alpha$-NH₂ acetylation of proteins is the most common co- and post-translational modification of soluble, intracellular proteins, and in some cells about 80% of their proteins are N$^\alpha$-acetylated (Brown, J.L. et al., J. Biol. Chem. 251:1009-1014 (1976); Brown, J.L., J. Biol. Chem. 254:1447-1449 (1979)) by N$^\alpha$-acetyltransferases utilizing acetyl-CoA. Although the presence of such N$^\alpha$-acetyltransferases has been widely demonstrated in both prokaryotic and eukaryotic cells, only recently has the first eukaryotic enzyme been purified and characterized (Lee, F.J.S. et al., J. Biol. Chem. 263:14948-14955 (1988)), and an encoding cDNA is now cloned and sequenced (Lee, F.J.S. et al., Proc. Natl. Acad. Sci. USA (submitted)). There are also numerous examples of covalent attachment of an acetyl group to biological substances other than proteins (e.g., glucosamine, choline, arylamine, arylalkyamine) (Klein, U. et al., Proc. Natl. Acad. Sci. USA 75:5185-5189 (1978); Roskoski, R., Jr., J. Biol. Chem. 249:2156-2159 (1974); Jencks, W.P. et al., J. Biol. Chem. 247:3756-3760 (1972); Weber, W. et al., Pharmacol. Rev. 37:25-79 (1985)). The concentration of acetyl-CoA in cells is primarily regulated by its rate of synthesis and its utilization in various metabolic pathways, and the role of acetyl-CoA hydrolase in regulating acetyl-CoA remains unclear. However, several functions for acetyl-CoA hydrolase have been proposed. First, the enzyme may be involved in the maintenance of the cytosolic acetyl-CoA concentration and CoASH pool for both fatty acid synthesis and oxidation (Prass, R.L. et al., J. Biol. Chem. 255:5215-5223 (1980)). Second, the mitochondrial enzyme may be involved in the metabolism of brown fat in hibernating animals (Bernson, V.S.M., Eur. J. Biochem. 67:403-410 (1976)). Third, the enzyme may be involved in the regulation of acetylation during melatonin synthesis in the pineal gland (Namboodiri, M.A.A. et al., J. Biol. Chem. 255:6032-6035 (1980)). Yet in no case has a specific biological function for this enzyme been firmly established.

## II. Genetic Engineering of Acetyl-CoA Hydrolase

This invention further comprises the amino acid sequences of acetyl-CoA hydrolase, the genetic sequences coding for the enzyme, vehicles containing the genetic sequence, hosts transformed therewith, enzyme production by transformed host expression, and utilization of the enzyme in the acetylation of peptides or proteins.

The DNA sequence coding for acetyl-CoA hydrolase may be derived from a variety of sources. For example, mRNA encoded for acetyl-CoA hydrolase may be isolated from yeast, or from other sources of the enzyme, by using the Northern blot method (Alwine et al., Method Enzymol.68:220-242 (1979)), and labeled oligonucleotide probes. The mRNA may then be converted to cDNA by techniques known to those skilled in the art. The probes may be synthesized based on the known amino acid sequences of acetyl-CoA hydrolase as described above.

Alternatively, degenerative DNA probes maybe used to screen a DNA library of a species that produces .acetyl-CoA hydrolase thereby isolating a clone that contains the DNA sequence encoding the enzyme. The DNA library is created by the fragmentation, using one or more restriction endonucleases of the genomic DNA, followed by incorporation into vectors, and use thereof to transform host cells, which are then plated and screened.

The DNA probe may be labeled with a detectable group. Such detectable group can be any material having a detectable physical or chemical property. Such materials have been well-developed in the field of immunoassays and in general almost any label useful in such methods can be applied to the present invention. Particularly useful are enzymatically active groups, such as enzymes (see Clin. Chem. 22:1243 (1976)), enzyme substrates (see British Pat. Spec. 1,548,741), coenzymes (see U.S. Pat. Nos. 4,230,797 and 4,238,565) and enzyme inhibitors (see U.S. Pat. No. 4,134,792); fluorescers (see Clin. Chem. 25:353 (1979)); chromophores; luminescers such as chemiluminescers and bioluminescers (see Clin. Chem. 25:512 (1979)); specifically bindable ligands; proximal interacting pairs; and radioisotopes such as $^3H$, $^{35}S$, $^{32}P$, $^{125}I$ and $^{14}C$. Such labels and labeling pairs are detected on the basis of their own physical properties (e.g., fluorescers, chromophores and radioisotopes) or their reactive or binding properties (e.g., enzymes, substrates, coenzymes and inhibitors). For example, a cofactor-labeled probe can be detected by adding the enzyme for which the label is a cofactor and a substrate for the enzyme. For example, one can use an enzyme which acts upon a substrate to generate a product with a measurable physical property. Examples of the latter include, but are not limited to, beta-galactosidase, alkaline phosphatase and peroxidase.

A DNA sequence encoding acetyl-CoA hydrolase may be recombined with vector DNA in accordance with conventional techniques, including blunt-ended or stagger-ended termini for ligation, restriction enzyme digestion to provide appropriate termini, filling in of cohesive ends as appropriate, alkaline phosphatase treatment to avoid undesirable joining, and ligation with appropriate ligases.

To express acetyl-CoA hydrolase, transcriptional and translational signals recognized by an appropriate host element are necessary. Eukaryotic hosts may be mammalian cells capable of culture in vitro, particularly leukocytes, more particularly myeloma cells or other transformed or oncogenic lymphocytes, e.g., EBV-transformed cells. Alternatively, non-mammalian cells may be employed, such as bacteria, fungi, e.g., yeast, filamentous fungi, or the like.

Possible hosts for acetyl-CoA hydrolase production are mammalian cells, grown in vitro in tissue culture or in vivo in animals. Mammalian cells may provide post-translational modifications to acetyl-CoA hydrolase molecules including correct folding or glycosylation of the correct sites. Mammalian cells which may be useful as hosts include cells of fibroblast origin such as VERO or CHO-K1, or cells of lymphoid origin, such as the hybridoma SP2/0-AG14 or the myeloma P3x63Sgh, and their derivatives. Usually the acetyl-CoA hydrolase construct will be part of a vector having a replication system recognized by the host cell.

In one embodiment, a procaryotic cell is transformed by a plasmid carrying the acetyl-CoA hydrolase-encoded gene. Bacterial hosts of particular interest include E. coli K12 strain 294 (ATCG-31446), E. coli X1776 (ATCC 31537), E. coli W3110 (F⁻, lambda⁻, phototropic (ATCC 27325)), and other enterobacterium such as Salmonella typhimurium or Serratia marcescens, and various Pseudomonas species. Under such conditions, the acetyl-CoA hydrolase acetyltransferase will not be glycosylated. The procaryotic host must be compatible with the replicon and control sequences in the expression plasmid.

In general, such vectors containing replicon and control sequences which are derived from species compatible with a host cell, are used in connection with the host. The vector ordinarily carries a replicon site, as well as specific genes which are capable of providing phenotypic selection in transformed cells. The expression of the acetyl-CoA hydrolase-encoded DNA can also be placed under control of other regulatory sequences which may be homologous to the organism in its untransformed state. For example, lactose-dependent E. coli chromosomal DNA comprises a lactose or lac operon which mediates lactose utilization by elaborating the enzyme β-galactosidase. The lac control elements may be obtained from bacteriophage lambda plac5, which is infective for E. coli. The lac promoter-operator system can be induced by IPTG.

Other promoter/operator systems or portions thereof can be employed as well. For example, colicin E1, galactose, alkaline phosphatase, tryptophan, xylose, tax, and the like can be used.

For a mammalian host, several possible vector systems are available for expression. One class of vectors utilize DNA elements which provide autonomously replicating extra-chromosomal plasmids, derived from animal viruses such as bovine papilloma virus, polyoma virus, adenovirus, or SV40 virus. A second class of vectors relies upon the integration of the desired gene sequences into the host chromosome. Cells which have stably integrated the introduced DNA into their chromosomes may be selected by also introducing one or markers which allow selection of host cells which contain the expression vector. The marker may provide for prototropy to an auxotrophic host, biocide resistance, e.g., antibiotics, or heavy metals, such as copper or the like. The selectable marker gene can either be directly linked to the DNA sequences to be expressed, or introduced into the same cell by co-transformation. Additional elements may also be needed for optimal synthesis of mRNA. These elements may include splice signals, as well as transcription promoters, enhancers, and termination signals. The cDNA expression vectors incorporating such elements include those described by Okayama, H., Mol. Cel. Biol. 3:280 (1983), and others.

A wide variety of transcriptional and translational regulatory sequences may be employed, depending on the nature of the host. The transcriptional and translational signals may be derived from viral sources, such as adenovirus, bovine papilloma virus, simian virus, or the like, where the regulatory signals are associated with a particular gene which has a high level of expression. Alternatively, promoters from mammalian expression products, such as actin, collagen, myosin, etc., may be employed. Transcriptional initiation signals may also be selected which allow for repression or activation, so that expression of the genes may be modulated. Of interest are regulatory signals which are temperature-sensitive so that varying the temperature, expression can be repressed or initiated, or are subject to chemical regulation, e.g., metabolite.

Once the vector or DNA sequence containing the constructs has been prepared for expression, the DNA constructs may be introduced to an appropriate host. Various techniques may be employed, such as protoplast fusion, calcium phosphate precipitation, electroporation or other conventional techniques. After the fusion, the cells are grown in media and screened for appropriate activities. Expression of the gene(s) results in production of the acetyl-CoA hydrolase.

The host cells for acetyl-CoA hydrolase production may be immortalized cells, primarily myeloma or lymphoma cells. These cells may be grown in a appropriate nutrient medium in culture flasks or injected into a synergistic host, e.g., mouse or rat, or immunodeficient host or host site, e.g., nude mouse or hamster pouch.

The acetyl-CoA hydrolase of the invention may be isolated and purified in accordance with conventional

14

conditions, such as extraction, precipitation, chromatography, affinity chromatography, electrophoresis, or the like.

## III. Uses of Acetyl-CoA Hydrolase

### Pharmaceutical Uses

Acetyl-CoA hydrolase once produced and purified, can be used, for example, in a pharmaceutical manufacturing environment to inhibit cellular acetylation. For example, the enzyme would inhibit acetylation of platelets, thus increasing clotting time.

### Expression of the Acetyl-CoA Hydrolase in Plants

Further, the acetyl-CoA hydrolase can be introduced into a plant by genetic engineering techniques, which upon production in the plant cell could be used as a means for blocking the acetylation of herbicides. Therefore, it is possible to produce a plant that is more herbicide-tolerant. In thus another embodiment of this invention, the acetyl-CoA hydrolase gene is used to transform a plant to enhance the herbicidal tolerance of the plant.

The coding region for a acetyl-CoA hydrolase gene that may be used in this invention may be homologous or heterologous to the plant cell or plant being transformed. It is necessary, however, that the genetic sequence coding for acetyl-CoA hydrolase be expressed, and produced, as a functional protein or polypeptide in the resulting plant cell. Thus, the invention comprises plants containing either homologous acetyl-CoA hydrolase genes or heterologous acetyl-CoA hydrolase genes that express the enzyme.

In one embodiment of this invention, the acetyl-CoA hydrolase comprises a plant acetyl-CoA hydrolase that is homologous to the plant to be transformed. In another embodiment of this invention, the acetyl-CoA hydrolase comprises an enzyme that is heterologous to the plant to be transformed. Moreover, DNA from both genomic DNA and cDNA encoding a acetyl-CoA hydrolase gene may be used in this invention. Further, a acetyl-CoA hydrolase gene may be constructed partially of a cDNA clone and partially of a genomic clone. In addition, the DNA coding for the acetyl-CoA hydrolase gene may comprise portions from various species.

There are a variety of embodiments encompassed in the broad concept of the invention. In one of its embodiments, this invention comprises chimeric genetic sequences:

(a) a first genetic sequence coding for a acetyl-CoA hydrolase that upon expression of the gene in a given plant cell is functional for acetyl-CoA hydrolase;

(b) one or more additional genetic sequences operably linked on either side of the acetyl-CoA hydrolase coding region. These additional genetic sequences contain sequences for promoter(s) or terminator(s). The plant regulatory sequences may be heterologous or homologous to the host cell.

In a preferred embodiment, the promoter of the acetyl-CoA hydrolase gene is used to express the chimeric genetic sequence. Other promoters that may be used in the genetic sequence include nos, ocs, and CaMV promoters. An efficient plant promoter that may be used is an overproducing plant promoter. This promoter in operable linkage with the genetic sequence for acetyl-CoA hydrolase should be capable of promoting expression of said acetyl-CoA hydrolase such that the transformed plant has increased tolerance to a herbicide. Overproducing plant promoters that may be used in this invention include the promoter of the small subunit (ss) of the ribulose-1,5-biphosphate carboxylase from soybean (Berry-Lowe et al., J. Molecular and App. Gen., 1:483-498 (1982), and the promoter of the chlorophyll a/b binding protein. These two promoters are known to be light induced in eukaryotic plant cells (see, for example, Genetic Engineering of Plants, an Agricultural Perspective, A. Cashmore, Plenum, New York 1983, pages 29-38; Corruzi, G. et al., J. of Biol. Chem., 258: 1399 (1983); and Dunsmuir, P. et al., J. of Mol. and Applied Genet., 2: 285 (1983)).

Further, in another preferred embodiment, the expression of the chimeric genetic sequence comprising the acetyl-CoA hydrolase gene is operably linked in correct reading frame with a plant promoter and with a gene secretion signal sequence.

The chimeric genetic sequence comprising a acetyl-CoA hydrolase gene operably linked to a plant promoter, and in the preferred embodiment with the secretion signal sequences, can be ligated into a suitable cloning vector. In general, plasmid or viral (bacteriophage) vectors containing replication and control sequences derived from species compatible with the host cell are used. The cloning vector will typically carry a replication origin, as well as specific genes that are capable of providing phenotypic selection markers in transformed host cells, typically resistance to antibiotics. The transforming vectors can be selected by these phenotypic markers after transformation in a host cell.

Host cells that may be used in this invention include procaryotes, including bacterial hosts such as E. coli, S. typhimurium, and Serratia marcescens. Eukaryotic hosts such as yeast or filamentous fungi may also be used in this invention.

The cloning vector and host cell transformed with the vector are used in this invention typically to increase the copy number of the vector. With an increased copy number, the vectors containing the acetyl-CoA hydrolase gene can be isolated and, for example, used to introduce the chimeric genetic sequences into the plant cells. The genetic material contained in the vector can be microinjected directly into plant cells by use of micropipettes to mechanically transfer the recombinant DNA. The genetic material may also be transferred into the plant cell by using polyethylene glycol which forms a precipitation complex with the genetic material that is taken up by the cell. (Paszkowski et al., EMBO J. 3:2717-22 (1984)).

In an alternative embodiment of this invention, the acetyl-CoA hydrolase gene may be introduced into the plant cells by electroporation. (Fromm et al., "Expression of Genes Transferred into Monocot and Dicot Plant Cells by Electroporation," Proc. Nat'l. Acad. Sci. U.S.A. 82:5824 (1985)). In this technique, plant protoplasts are electroporated in the presence of plasmids containing the acetyl-CoA hydrolase genetic construct. Electrical impulses of high field strength reversibly permeabilize biomembranes allowing the introduction of the plasmids. Electroporated plant protoplasts reform the cell wall, divide, and form plant callus. Selection of the transformed plant cells with the expressed acetyl-CoA hydrolase can be accomplished using the phenotypic markers as described above.

Another method of introducing the acetyl-CoA hydrolase gene into plant cells is to infect a plant cell with Agrobacterium tumefaciens transformed with the acetyl-CoA hydrolase gene. Under appropriate conditions known in the art, the transformed plant cells are grown to form shoots, roots, and develop further into plants. The acetyl-CoA hydrolase genetic sequences can be introduced into appropriate plant cells, for example, by means of the Ti plasmid of Agrobacterium tumefaciens. The Ti plasmid is transmitted to plant cells on infection by Agrobacterium tumefaciens and is stably integrated into the plant genome. Horsch et al., "Inheritance of Funtional Foreign Genes in Plants," Science 233:496-498 (1984); Fraley et al., Proc. Nat'l Acad. Sci. U.S.A. 80:4803 (1983)).

Ti plasmids contain two regions essential for the production of transformed cells. One of these, named transfer DNA (T DNA), induces tumor formation. The other, termed virulent region, is essential for the formation but not maintenance of tumors. The transfer DNA region, which transfers to the plant genome, can be increased in size by the insertion of the enzyme's genetic sequence without its transferring ability being affected. By removing the tumor-causing genes so that they no longer interfere, the modified Ti plasmid can then be used as a vector for the transfer of the gene constructs of the invention into an appropriate plant cell.

All plant cells which can be transformed by Agrobacterium and whole plants regenerated from the transformed cells can also be transformed according to the invention so to produce transformed whole plants which contain the transferred acetyl-CoA hydrolase gene.

There are presently two different ways to transform plant cells with Agrobacterium:

(1) co-cultivation of Agrobacterium with cultured isolated protoplasts, or

(2) transforming cells or tissues with Agrobacterium.

Method (1) requires an established culture system that allows culturing protoplasts and plant regeneration from cultured protoplasts.

Method (2) requires (a) that the plant cells or tissues can be transformed by Agrobacterium and (b) that the transformed cells or tissues can be induced to regenerate into whole plants. In the binary system, to have infection, two plasmids are needed: a T-DNA containing plasmid and a vir plasmid.

After transformation of the plant cell or plant, those plant cells or plants transformed by the Ti plasmid so that the enzyme is expressed, can be selected by an appropriate phenotypic marker. These phenotypical markers include, but are not limited to, antibiotic resistance. Other phenotypic markers are known in the art and may be used in this invention.

All plants from which protoplasts can be isolated and cultured to give whole regenerated plants can be transformed by the present invention so that whole plants are recovered which contain the transferred acetyl-CoA hydrolase gene. Some suitable plants include, for example, species from the genera Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum, Sorghum, and Datura.

There is an increasing body of evidence that practically all plants can be regenerated from cultured cells or tissues, including but not limited to all major cereal crop species, sugarcane, sugar beet, cotton, fruit and other trees, legumes and vegetables. Limited knowledge presently exists on whether all of these plants can be transformed by Agrobacterium. Species which are a natural plant host for Agrobacterium may be transformable in vitro. Monocotyledonous plants, and in particular, cereals and grasses, are not natural hosts to Agrobacterium. Attempts to transform them using Agrobacterium have been unsuccessful until recently. Hooykas-Van Slogteren et al., Nature 311:763-764 (1984). There is growing evidence now that certain monocots can be transformed by Agrobacterium. Using novel experimental approaches that have now become available, cereal and grass species may be transformable.

Additional plant genera that may be transformed by Agrobacterium include Ipomoea, Passiflora, Cyclamen, Malus, Prunus, Rosa, Rubus, Populus, Santalum, Allium, Lilium, Narcissus, Ananas, Arachis, Phaseolus, and Pisum.

Plant regeneration from cultural protoplasts is described in Evans et al., "Protoplast Isolation and Culture," in Handbook of Plant Cell Culture 1:124-176 (MacMillan Publishing Co., New York, 1983); M.R. Davey, "Recent Developments in the Culture and Regeneration of Plant Protoplasts." Protoplasts, 1983 - Lecture Proceedings, pp. 19-29 (Birkhauser, Basel, 1983); P.J. Dale, "Protoplast Culture and Plant Regeneration of Cereals and Other Recalcitrant Crops," in Protoplasts 1983 - Lecture Proceedings, pp. 31-41 (Birkhauser, Basel, 1983); and H. Binding, "Regeneration of Plants", in Plant Protoplasts, pp. 21-37 (CRC Press, Boca Raton, 1985).

Regeneration varies from species to species of plants, but generally a suspension of transformed protoplasts containing multiple copies of the acetyl-CoA hydrolase gene is first provided. Embryo formation can then be induced from the protoplast suspensions, to the stage of ripening and germination as natural embryos. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

The mature plants, grown from the transformed plant cells, are selfed to produce an inbred plant. The inbred plant produces seed containing the gene for the increased acetyl-CoA hydrolase. These seeds can be grown to produce plants that have enhanced acetyl-CoA hydrolase which will block the acetylation of herbicides.

The inbreds according to this invention can be used to develop herbicide tolerant hybrids. In this method, a herbicide tolerant inbred line is crossed with another inbred line to produce the hybrid.

Parts obtained from the regenerated plant, such as flowers, sees, leaves, branches, fruit, and the like are covered by the invention provided that these parts comprise the herbicidal tolerant cells. Progeny and variants, and mutants of the regenerated plants are also included within the scope of this invention.

In diploid plants, typically one parent may be transformed by the acetyl-CoA hydrolase acetyltransferase genetic sequence and the other parent is the wild type. After crossing the parents, the first generation hybrids (F1) will show a distribution of 1/2 acetyl-CoA hydrolase/wild type:1/2 acetyl-CoA hydrolase/wild type. These first generation hybrids (F1) are selfed to produce second generation hybrids (F2). The genetic distribution of the F2 hybrids are 1/4 acetyl-CoA hydrolase/acetyl-CoA hydrolase : 1/2 acetyl-CoA hydro lase/wild type : 1/4 wild type/wild type. The F2 hybrids with the genetic makeup of acetyl-CoA hydrolase/acetyl-CoA hydrolase are chosen as the herbicidal tolerant plants.

As used herein, variant describes phenotypic changes that are stable and heritable, including heritable variation that is sexually transmitted to progeny of plants, provided that the variant still comprises a herbicidal tolerant plant through enhanced rate of acetylation. Also, as used herein, mutant describes variation as a result of environmental conditions, such as radiation, or as a result of genetic variation in which a trait is transmitted meiotically according to well-established laws of inheritance. The mutant plant, however, must still exhibit a herbicidal tolerance through inhibition of herbicidal acetylation as according to the invention.

## EXAMPLES

## Materials and Methods

### Materials

The following abbreviations are used herein: A buffer, 50 mM Tris-HCl, pH 7.8, 10 mM MgCl$_2$, 3 mM DTT, 1 M sorbitol; acetyl-CoA, acetyl coenzyme A; B buffer, 10 mM HEPES, pH 7.4, 1.5 mM MgCl$_2$, 10 mM KCl, and 0.5 mM DTT; CHES, 2-(N-cyclohexylamino) ethane-sulfonic acid; CoASH, coenzyme A; DEPC, diethyl pyrocarbonate; HDG buffer, 20 mM HEPES, pH 7.4, 0.5 mM DTT, 10% (v/v) glycerol and 0.02% NaN$_3$; HEPES, N-2-hydroxyethylpiperazine-N'-2-ethanesulfonic acid; HNBS(CH$_3$)$_2$-Br, dimethyl-(2-hydroxy-5-nitrobenzyl)sulfonium bromide; IAA, iodoacetic acid; IAM, iodoacetamide; NBS, N-bromosuccinimide; NEM, N-ethylmaleimide; pCMB, p-chloromercuribenzoate; SDS-PAGE, sodium dodecyl sulfate-polyacryamide gel electrophoresis; TNBS, 2,4,6-trinitrobenzenesulfonic acid.

NEM, IAA, IAM, HNBS(CH$_3$)$_2$-Br, TNBS N-acetylimidazole, pCMB, NBS, DEPC, acetoacetyl-CoA, butyryl-CoA, malonyl-CoA, propionyl-CoA, myristoyl-CoA, HEPES, CAPS, CHES, DTT, hydroxylamine, bovine serum albumin, protein standards for M$_r$ determinations, 2-mercaptoethanol, glucose, sorbitol, lyticase, Bit-Tris, Tris and glycerol (enzyme grade) were from Sigma. DEAE-Sepharose CL-6B, Sepharose CL-4B and chloramphenicol acetyltransferase were from Pharmacia. Protein assay reagent (Bradford method), hydroxylapatite (Biogel HT), Affi-Gel Blue gel and SDS-PAGE electrophoresis reagents were from BioRad. [$^3$H] Acetyl coenzyme A was from Amersham, and unlabeled acetyl coenzyme A was from P-L Biochemicals. Reagents and solvents from amino acid analysis and Ready-Solv EP scintillation cocktails were obtained from Beckman. SP membrane was from Cuno Inc. PM-30 membrane was from Amicon. Yeast extract and Bacto-peptone were from Difco. Constant boiling (6 N) HCl and Polybrene were from Pierce. Phenol was from BRL. Microdialyzer was from Health Products. Reagents and solvents for protein sequence analysis were from Applied Biosystems. Reagents for peptide synthesis were obtained from Applied Biosystems, and solvents for peptide synthesis were from Anachem. Boc-amino acids were from Peninsula. All other chemicals were reagent grade or better.

### Methods

#### General

UV measurements were obtained using a Hewlett-Packard 8450A UV spectrophotometer. Protein assays were performed by the method of Bradford (Anal. Biochem 72:248-254 (1976)) using bovine serum albumin as the standard. Radioactive samples were counted on a Beckman LS 3801 scintillation counter.

#### Assays for Acetyl-CoA Hydrolase Activity

##### A. Acetylation Inhibition Assay

The principle of this assay is that acetyl-CoA hydrolase will hydrolyse acetyl-CoA and thereby deplete the amount of acetyl-CoA from which a labeled acetyl group may be transferred by N$^\alpha$-acetyltransferase to ACTH (1-24). The assay for inhibition of acetylation uses a previously described assay for N$^\alpha$-acetyltransferase (Lee, F.J.S., et al., J. Biol. Chem. 263:14948-14955 (1988)) and is carried out as follows: aliquots of acetyl-CoA hydrolase solutions (2-20 µl) were added to 1.5 ml Eppendorf tubes containing a reaction mixture of 50 mM HEPES, pH 7.4, 150 mM KCl, 1 mM DTT, 50 µM ACTH (1.24), and partial purified yeast N$^\alpha$-acetyltransferase from the hydroxylapatite chromatography step (Lee, F.J.S., et al., J. Biol. Chem. 263:14948-14955 (1988)) (~10 units) in a final volume of 100 µl. The reaction was initiated by adding 25 µM [$^3$H] acetyl-CoA (0.5 µCi). The assay mixture was incubated at 30°C for 30 min. The reaction was stopped by adding 17 µl of 0.5 M acetic acid and by chilling in an ice bath. The stopped reaction mixtures were filtered through 2.5 cm diameter SP membrane discs. The membranes were washed three times with 1 ml of 0.5 M acetic acid to remove the free [$^3$H] acetyl-CoA. The partially dried membranes were placed in 10 ml of Ready-Solv EP scintillation cocktail and counted for 1 min.

The number of acetyl-CoA hydrolase units is calculated as follows:

$$cpm_{ACT} - cpm_{ACT} + ACH$$

--------------------------------

$$cpm_{ACT} - cpm_{BKD} / 10 \text{ (units)}$$

where $cpm_{ACT}$ is the number of counts when 10 units of $N^{\alpha}$-acetyltransferase alone is present; $cpm_{ACT + ACH}$ is the number of counts when 10 units of $N^{\alpha}$-acetyltransferase as well as an unknown amount of acetyl-CoA hydrolase are present; and $cpm_{BKD}$ is the number of background counts. Calculation of the number of units of acetyl-CoA hydrolase is carried out when the value of $cpm_{ACT + ACH}$ equals approximately 50% of the value of $cpm_{ACT}$.

One unit of acetyl-CoA hydrolase activity is defined as the amount of enzyme able to inhibit 1 unit of $N^{\alpha}$-acetyltransferase activity. One unit of $N^{\alpha}$-acetyltransferase activity is defined as the amount of enzyme able to transfer 1 pmol of [³H] acetyl group from [³H] acetyl-CoA to ACTH (1-24) (Lee, F.J.S., et al., J. Biol. Chem. 263:14948-14955 (1988)).

(B) Radioactive Assay

The principle of the assay is that free acetate, but not acetyl-CoA, will evaporate from an acid solution at an elevated temperature. Aliquots of enzyme solution (1-10 $\mu$l) were added to 0.5 ml Eppendorf tubes containing a reaction mixture of 100 mM potassium phosphate buffer, pH 7.4 and 0.25 mM [1-¹⁴C] acetyl-CoA (0.5 Ci/mol) in a final volume of 100 $\mu$l. The assay mixture was incubated at 30° C for 0.30 s, 1 min, 2 min, 3 min, 4 min, and 5 min. The reaction was stopped by adding 20 $\mu$l of 10 M acetic acid. The sample was evaporated by $N_2$ stream at 30° C to dryness in a fume hood, dissolved in 100 $\mu$l $H_2O$, and transferred to a scintillation vial containing 10 ml Ready-Solv EP scintillation cocktail. The sample tube was washed twice with 100 $\mu$l $H_2O$, each wash was transferred to the scintillation vial, and the radioactivity was determined by scintillation counting for 1 min. The radioactivity in the control without added acetyl-Coa hydrolase was subtracted from the radioactivity determined at each time point. One unit of activity is defined as the amount of enzyme which hydrolyzes 1 nmol [1-¹⁴C] acetyl-CoA in 1 minute.

(C) Colorimetric Assay

In the substrate specificity experiment, enzyme activity is measured by following an increase in $A_{412}$ ($\Sigma = 13,600$ M⁻¹) when free CoASH generated during deacylation of acetyl-CoA reacts with 5,5'-dithio-bis(2-nitro-benzoic acid) (DNTB), as previously described (Robinson, J.B., et al, Biochem. Biophys. Res. Commun. 71:959-965 (1976); Bernson, V.S.M., Eur. J. Biochem. 67:403-410 (1976)). Each assay solution contained: Tris/HCl, pH 7.6, 100 mM; DTNB, 0.4 mM; acetyl CoA, 0.25 mM, and an aliquot of an acetyl-CoA hydrolase solution was added in a final volume of 1 ml. The reaction was started by the addition of the aliquot of an acetyl-CoA hydrolase solution, the reaction mixture was incubated at 30° C, and the $\Delta A_{412}$ was measured at 0.30 s, 1 min, 2 min, 3 min, 4 min, and 5 min using a Hewlett-Packard 8450A diode array spectrophotometer.

Cell Growth and Storage

A bakers' yeast strain (TD 71.8) was grown aerobically at 30° C in YPD medium (1% yeast extract, 2% Bacto-peptone, 2% glucose) in a Chemap AG fermentor (Chemap AG, Volketswil, Switzerland). Cells were harvested when the culture reached on $OD_{660 \text{ nm}}$ of 14, concentrated to 38 liters by Alfa-Laval separation system (Alfa-Laval Separation AB, Tumba, Sweden), and stored at -20° C with 10% (v/v) glycerol for up to 4 months without loss of activity.

Cell Extraction

Concentrated yeast culture (6 liters) was thawed, and yeast cells were collected by centrifugation at 4,000 rpm for 10 min (JS-4.0 rotor, Beckman). The cells (800 g, wet weight) were resuspended in 1 liter of A buffer (50 mM Tris-HCl, pH 7.8, 10 mM $MgCl_2$, 3 mM DTT, 1 M sorbitol) containing 80 mg of lyticase, and the cell suspension was shaken gently at 30°C for 45 min. All subsequent steps were carried out at 4°C. The spheroplasts were collected by centrifugation at 4,000 rpm for 10 min (JS-4.0 rotor, Beckman), washed by gentle resuspension in 500 ml of A buffer, collected by centrifugation and resuspended gently in 400 ml of B buffer (10 mM HEPES, pH 7.4, 1.4 mM $MgCl_2$, 10 mM KCl, and 0.5 mM DTT). The spheroplasts were lysed in this hypotonic buffer by 15 strokes with a tight-fitting pestle and 15 strokes with a loose-fitting pestle in a Dounce homogenizer, and then cold KCl (2.0 M) was added to give a final KCl concentration of 0.2 M. The homogenate was gently shaken for 30 min, and debris removed by centrifugation at 14,000 rpm for 34 min (JA-14 rotor, Beckman). the supernatant solution was concentrated to a volume of 60 ml, using a PM-30 ultrafiltration membrane and dialyzed overnight against 8 liters of HDG buffer (20 mM HEPES, pH 7.4, 0.5 mM DTT, 10% (v/v) glycerol, and 0.02% $NaN_3$) containing 0.2 M KCl.

### DEAE-Sepharose CL-6B Chromatography

DEAE-Sepharose CL-6B was prepared, degassed, and packed into two different columns (2.5 x 55 cm), following the manufacturer's recommendations. The columns were washed with 4 column volumes of HDG buffer containing 0.2 M KCl (for 0.2 M KCl chromatography) or 0.05 M KCl (for linear KCl gradient chromatography). The dialyzed supernatant solution was applied to DEAE-Sepharose CL-6B equilibrated with HDG buffer containing 0.2 M KCl. Both $N^\alpha$-acetyltransferase and acetyl-CoA hydrolase activity co-eluted from this DEAE-Sepharose (CL-6B column at a flow rate of 24 ml/h, as previously shown (Lee, F.J.S., et al., J. Biol. Chem. 263:14948-14955 (1988)). Fractions (4 ml) were collected, and the fractions containing $N^\alpha$-acetyltransferase and acetyl-CoA hydrolase activity were pooled and concentrated to a volume of 50 ml, using a PM-30 ultrafiltration membrane. This concentrated eluate was dialyzed overnight against 2 x 4 liters of HDG buffer containing 0.05 M KCl and then applied to a second DEAE-Sepharose CL-6B column (2.5 x 55 cm) equilibrated in HDG buffer containing 0.5 M KCl. This column was eluted with a linear gradient of 0.05 M (250 ml) to 0.5 M (250 ml) KCl in HDG buffer at 24 ml/h. Fractions (3.6 ml) were collected, and the fractions containing acetyl-CoA hydrolase activity were pooled (Figure 1) and concentrated to a volume of 5 ml, using a PM-30 ultrafiltration membrane.

### Sepharose CL-4B Chromatography

The concentrated eluate from the second DEAE-Sepharose chromatography was applied to a Sepharose CL-4B column (2.5 x 96 cm). The column was eluted with HDG buffer containing 0.2 M KCl at 20 ml/h. The elution volume of the enzyme was determined by $A_{280\ nm}$ and enzyme activity. Fractions (3.0 ml) were collected, and the fractions containing acetyl-CoA hydrolase activity were pooled (Figure 2) and concentrated to a volume of 1.5 ml, using a PM-30 ultrafiltration membrane.

### Hydroxylapatite Chromatography

The concentrated eluate from Sepharose CL-4B chromatography was dialyzed overnight against 2 x 4 liters of 0.05 M potassium phosphate buffer, pH 74, 0.5 mM DTT, 10% (v/v) glycerol, 0.02% $NaN_3$ and applied to a hyroxylapatite column (2.0 x 25 cm) equilibrated with the same buffer used for dialysis. The column was eluted with a linear gradient of 0.05 M (150 ml) to 0.5 M (150 ml) potassium phosphate buffer, pH 7.4, containing 0.5 mM DTT, 10% (v/v) glycerol, 0.02% $NaN_3$ at 12 ml/h. Fractions (1.8 ml) were collected, and the fractions containing acetyl-CoA hydrolase activity were pooled (Figure 3) and concentrated to a volume of 1.5 ml, using a PM-30 ultrafiltration membrane. Aliquots (25 $\mu$l) of various fractions from fractions 40-60 were analyzed on a SDS-PAGE gel (9%) under reducing conditions, as described by Laemmli (Laemmli, U.K., Nature 227:680-685 (1970)). Protein bands were stained with Coomassie Brilliant Blue R.

### Sodium Dodecyl Sulfate-Polyacrylamide Gel Electrophoresis

20

A sample of each pool containing acetyl-CoA hydrolase isolated from each purification step was analyzed on a SDS-PAGE gel (9%) under reducing conditions, as described by Laemmli (Laemmli, U.K., Nature 227:680-685 (1970)). For determination of $M_r$ of purified enzyme subunits, myosin (205,000), E. coli β-galactosidase (116,000), rabbit muscle phosphorylase (97,000), bovine serum albumin (66,000), egg albumin (45,000), and carbonic anhydrase (29,000) were used as molecular weight standards. Protein bands were stained with Coomassie Brilliant Blue R. Higher $M_r$ bands were not observed in lanes containing either crude extract or the DEAE-Sepharose (0.2 M KCl) pool The absence of such bands is due to proteolytic degradation which occurred during prolonged storage.

Isoelectric Point Determination

Isoelectric focusing, using an aliquot from the hydroxylapatite pool (Figure 3), was performed as previously described (Catsimpoolas, N., Isoelectric Focusing, Academic Press, New York (1976)) (pH range 3.5-9.5). For determination of pI of purified enzyme subunits, A. oryzae amyloglucosidase (pI = 3.55), soybean trypsin inhibitor (pI = 4.55), milk β-lactoglobulin (pI - 5.13), bovine erythrocytes carbonic anhydrase B (pI = 5.85), human erythrocytes carbonic anhydrase B (pI = 6.57), horse heart myoglobin (pI = 6.76 and 7.16), rabbit muscle L-lactic dehydrogenase (pI = 8.3, 8.4, and 8.55), and bovine pancreas trypsinogen (pI = 9.3) were used as pI standards. Protein bands were stained with Coomassie Brilliant Blue R.

Molecular Size Determination

The $M_r$ of the native protein was estimated by comparison to molecular weight standards by gel filtration on Sepharose CL-4B column (2.5 x 96 cm). An aliquot of the purified enzyme from the hydroxylapatite chromatography pool was applied to the column. The column was eluted with HDG buffer containing 0.2 M KCl at 20 ml/h. The elution volume of the enzyme was determined by $A_{280\ nm}$ and enzyme activity (acetylation inhibition assay), and the apparent molecular weight of yeast acetyl-CoA hydrolase was calculated by comparison with the relative elution volumes of protein standards including thyroglobulin (669,000), apoferritin (443,000), β-amylase (200,000), alcohol dehydrogenase (150,000), bovine serum albumin (66,000) and carbonic anhydrase (29,000).

Amino Acid Analysis

The concentrated eluate from the hydroxylapatite chromatography was applied to a 7% SDS-PAGE gel of 1.5 mm thickness in a 12 cm well, electrophoresed, and electroeluted as previously described (Hunkapiller, M., et al., Methods Enzymol. 91:227-236 (1983)). The amino acid composition was determined using a Beckman 6300 Amino Acid Analyzer after 24 hr hydrolysis at 110°C in 6 N HCl containing 0.1% phenol (Moore, S., Chemistry and Biology of Peptides, Meienhofer, J., ed., Ann Arbor Science, Ann Arbor, MI, pp. 629-652).

Protein Sequence Analysis

Protein sequence analysis of electroeluted acetyl-CoA hydrolase was carried out twice (each ~300 pmole) by using an Applied Biosystems 470A Protein Sequencer and an Applied Biosystems 120A Pth Analyzer (Hewick, R.M., et al., J. Biol. Chem. 256:7990-7997 (1981)).

Chemical Modifications

DEPC (liquid, approximately 6.8 M) was diluted to 1 M with cold 100% ethanol. Further dilutions of DEPC were made with 0.1 M sodium phosphate (pH 6.0) containing 1 mM EDTA and 5% ethanol. NBS and succinic anhydride were dissolved in acetone and diluted with distilled water prior to use. NEM, IAA, IAM, (HNBS(CH$_3$)$_2$-Br), TNBS, and N-acetylimidazole solution were prepared in distilled water. pCMB was prepared in 10 mM NaOH as a concentrated solution and diluted with distilled water prior to use.

The individual inhibition experiments were done at 25°C by incubating the enzyme in 100m·mM potassium phosphate buffer (pH 7.4) containing the various modifications reagents, except for (HNBS(CH₃)-2-Br) and DEPC, which were done in 50 mM sodium phosphate buffer (pH 6.0). After 15 min, each sample aliquot was dialyzed using a microdialyzer against 100 mM potassium phosphate, pH 7.4, 1 mM DTT at 4°C for 3 to 40 h. The enzyme activity was determined using the radioactive assay for acetyl-CoA hydrolase. As a control, the enzyme was incubated separately without added reagents.

Peptide Synthesis

Human ACTH (1-24) were synthesized on an Applied Biosystems Model 430A peptide synthesizer and characterized, as previously described (Finnegan, A., et al., J. Exp. Med. 164:897-910 (1986)).

Although the foregoing refers to particular preferred embodiments, it will be understood that the present invention is not so limited. It will occur to those ordinarily skilled in the art that various modifications may be made to the disclosed embodiments and that such modifications are intended to be within the scope of the present invention.

Claims

1. A substantially purified acetyl-CoA hydrolase having the following characteristics;
(a) a molecular weight of about 64,000 ± 5,000 daltons;
(b) isoelectric point at about 5.8;
(c) pH optimum at 8.0;
(d) maximum activity at temperatures from 37° to 42°C wherein one unit of acetyl CoA hydrolase activity is defined as the amount of enzyme able to inhibit 1 unit of acetyltransferase activity wherein 1 unit of acetyltransferase activity is defined as the amount of enzyme able to transfer 1 pmol of [3H] acetyl group from [3H] acetyl coenzyme A to ACTH (1-24) by acetyltransferase standard enzyme assay under standard conditions;
(e) irreversible denaturation occurring after 1 minute at 70 C;
(f) substrate specificity dependent; and
(g) inactivation by chemical modification with diethyl pyrocarbonate.

2. The acetyl-CoA hydrolase of claim 2 having enzyme activity greater than 20, preferably greater than 50, more preferably greater than 1000, especially greater than 3000, wherein one unit of acetyl CoA hydrolase activity is defined as the amount of enzyme able to inhibit 1 unit of acetyltransferase activity wherein 1 unit of acetyltransferase activity is defined as the amount of enzyme able to transfer 1 pmol of [3H] acetyl group from [3H] acetyl coenzyme A to ACTH (1-24) by acetyltransferase standard enzyme assay under standard conditions.

3. A method of recovering substantially purified acetyl-CoA hydrolase from a sample containing acetyl-CoA hydrolase comprising the following steps:
(a) recovering crude acetyl-CoA hydrolase from a sample containing said acetyl-CoA hydrolase;
(b) subjecting said crude acetyl-CoA hydrolase from step (a) to ion exchange to obtain active fractions of acetyl-CoA hydrolase wherein one unit of acetyl-CoA hydrolase activity is defined as the amount of enzyme able to inhibit 1 unit of acetyltransferase activity wherein 1 unit of acetyltransferase activity is defined as the amount of enzyme able to transfer 1 pmol of [3H] acetyl group from [3H] acetyl coenzyme A to ACTH (1-24) by acetyltransferase standard enzyme assay under standard conditions; and
(c) applying said active fractions of acetyl-CoA hydrolase from step (b) to adsorption hydroxyapatite chromatography to obtain substantially purified acetyl-CoA hydrolase.

4. Acetyl-CoA hydrolase with a molecular weight of about 64,000 daltons, said acetyl-CoA hydrolase having an enzyme activity greater than 1000 wherein one unit of acetyl CoA hydrolase activity is defined as the amount of enzyme able to inhibit 1 unit of acetyltransferase activity under standard conditions, obtainable by a process comprising the steps of:
(a) recovering crude acetyl-CoA hydrolase from a sample containing said acetyl-CoA hydrolase;
(b) subjecting said crude acetyl-CoA hydrolase from step (a) to ion exchange to obtain active fractions of acetyl-CoA hydrolase wherein one unit of acetyl CoA hydrolase activity is defined as the amount of enzyme able to inhibit 1 unit of acetyltransferase activity wherein 1 unit of acetyltransferase activity is defined as the amount of enzyme able to transfer 1 pmol of [3H] acetyl group from [3H] acetyl coenzyme A to ACTH (1-24) by acetyltransferase standard enzyme assay under standard conditions; and

(c) applying said active fractions of acetyl-CoA hydrolase from step (b) to adsorption hydroxyapatite chromatography to obtain substantially purified acetyl-CoA hydrolase.

5. A peptide fragment of acetyl-CoA consisting essentially of the following amino acid sequence: Phe-Asn-Leu-Phe-Val-Gly-Ala-Ser-Ala-Gly-Pro-Glu-Glu-Asn-(Arg).

6. A peptide fragment of acetyl-CoA consisting essentially of the following amino acid sequence: Val-Val-Ala-Ile-Val-Glu-Ser-Thr-Met-Arg-Leu.

7. A recombinant DNA molecule comprising a genetic sequence coding for acetyl-CoA hydrolase.

8. A plant comprising cells transformed by the genetic sequence encoding the recombinant DNA molecule of Claim 7.

9. A method of producing a plant with increased herbicidal tolerance comprising transforming a plant with the genetic sequence encoding the recombinant DNA molecule of claim 7.

Claims for the following Contracting State: ES

1. A method for recovering substantially purified acetyl-CoA hydrolase from a sample containing acetyl-CoA hydrolase comprising the following steps:

(a) recovering crude acetyl-CoA hydrolase from a sample containing said acetyl-CoA hydrolase;

(b) subjecting said crude acetyl-CoA hydrolase from step (a) to ion exchange to obtain active fractions of acetyl-CoA hydrolase wherein one unit of acetyl CoA hydrolase activity is defined as the amount of enzyme able to inhibit 1 unit of acetyltransferase activity wherein 1 unit of acetyltransferase activity is defined as the amount of enzyme able to transfer 1 pmol of [$^3$H] acetyle group from [$^3$H] acetyl coenzyme A to ACTH (1-24) by acetyltransferase standard enzyme assay under standard conditions; and

(c) applying said active fractions of acetyl-CoA hydrolase from step (b) to adsorption hydroxyapatite chromatography to obtain substantially purified acetyl-CoA hydrolase.

2. A method as claimed in claim 1, wherein a substantially purified acetyl-CoA hydrolase is recovered having the following characteristics;

(a) a molecular weight of about 64,000 ± 5,000 daltons;

(b) isoelectric point at about 5.8;

(c) pH optimum at 8.0;

(d) maximum activity at temperatures from 37° to 42°C;

(e) irreversible denaturation occurring after 1 minute at 70 C;

(f) substrate specificity dependent; and

(g) inactivation by chemical modification with diethyl pyrocarbonate.

3. A method as claimed in claim 1, wherein an acetyl-CoA hydrolase is recovered having enzyme activity greater than 20.

4. A method as claimed in claim 1, wherein an acetyl-CoA hydrolase is recovered having enzyme activity greater than 50.

5. A method as claimed in claim 1, wherein an acetyl-CoA hydrolase is recovered having enzyme activity greater than 1,000.

6. A method as claimed in claim 1, wherein an acetyl-CoA hydrolase is recovered having enzyme activity greater than 3000.

7. A method of producing a plant with increased herbicidal tolerance, comprising transforming a plant with the genetic sequence encoding the acetyl-CoA-hydrolase obtainable according to claim 1.

FIGURE 1

## FIGURE 2

FIGURE 3

FIGURE 4

FIGURE 5 A

FIGURE 5 B

Data from "InhAcCoA.Km"

FIGURE 6 A

EP 0 348 958 A2

FIGURE 6 B

Data from "InhAcCoA.Km"

$y = 22.4484 + 1384.3788x$   $R = 0.99$

Km of Ac-CoA = 61.7 uM

1/S [uM]

1/V